# FASCICULE DE BREVET EUROPEEN

(11) **EP 0 725 058 B1**
(45) Date de publication et mention de la délivrance du brevet: **15.01.1997**
(21) Numéro de dépôt: 95119957.9
(22) Date de dépôt: 18.12.1995
(51) Int. Cl.: C07C 279/14, A61K 7/021

(54) **Nouveaux dérivés d'arginine, procédé de préparation, utilisations et compositions les comprenant**
Arginin-Derivate, Herstellungsverfahren, ihre Verwendung und Zusammensetzungen, die sie enthalten
Arginine derivatives, process for their preparation, their use and compositions containing them

(30) Priorité: 31.01.1995 FR 9501114
(43) Date de publication de la demande: 07.08.1996
(73) Titulaire: L'OREAL, 75008 Paris (FR)
(72) Inventeur: Bordier, Thierry, F-93290 Tremblay-en-France (FR); Genard, Sylvie, F-75012 Paris (FR); Philippe, Michel, F-91320 Wissous (FR)
(74) Mandataire: Dodin, Catherine

(56) Documents cités:
- EP-A- 0 139 481
- EP-A- 0 336 265
- US-A- 3 856 848
- US-A- 4 477 429

## Description

La présente invention a pour objet des composés à groupement Nα-alkyloxycarbonyle, dérivés de l'arginine, leur procédé de préparation, leur utilisation notamment en cosmétique et les compositions, notamment cosmétiques, les comprenant.

On connaît des compositions, notamment cosmétiques, pharmaceutiques ou alimentaires, qui peuvent se présenter sous forme d'une poudre dite compacte, obtenue par compactage. Il s'agit généralement de compositions anhydres pouvant être constituées principalement de particules solides et d'un liant gras, mises en forme par compression.
L'élaboration de telles compositions soulève toutefois de nombreuses difficultés car la composition finale doit être suffisamment homogène et compacte pour présenter une bonne aptitude au prélèvement et pour éviter par ailleurs une fragmentation pouvant être provoquée notamment par des chocs.

Il est décrit, dans la demande de brevet EP139481, des compositions cosmétiques utilisant comme agents pour modifier la surface de composés inorganiques, en vue d'en augmenter la dispersibilité, soit un dérivé monoacylé d'un acide aminé basique dont le groupement acyle aliphatique a 8-22 atomes de carbone, soit un dérivé N,N-diacylé d'un acide aminé basique dont les groupements acyles identiques ou différents, ont 1-22 atomes de carbone.
Il est également décrit, dans la demande de brevet EP336265, des compositions cosmétiques pour la mise en forme des cheveux comprenant comme agents tensioactifs, un dérivé N-monoacylé d'un acide aminé basique dont le groupement acyle a 8-22 atomes de carbone.
On constate toutefois que les dérivés acylés des acides aminés basiques décrits précédemment sont très difficilement compactables, voire intassables.

L'invention a pour but de proposer de nouveaux composés, qui permettent de faciliter l'obtention de telles compositions, tout en satisfaisant aux exigences précitées, sans présenter les inconvénients de l'art antérieur.

La présente invention a donc pour objet un dérivé d'arginine à groupements N^{α}-alkyloxycarbonyle de formule (I) 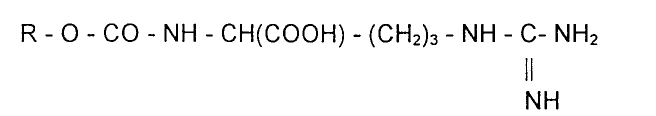
dans laquelle R représente un radical alkyle saturé, linéaire ou ramifié ayant de 8 à 22 atomes de carbone,
les sels des composés de formule (I), leurs isomères optiques de configuration D ou L, et leurs mélanges.

Un autre objet de l'invention est une composition, entre autre cosmétique, pharmaceutique, hygiénique ou alimentaire, comprenant au moins un dérivé de formule (I).
Encore un objet de l'invention est l'utilisation d'au moins un dérivé de formule (I) comme substance de revêtement de particules substrat. On a en effet constaté que lesdites particules, généralement des poudres, lorsqu'elles étaient enrobées par le dérivé selon l'invention, permettent d'obtenir, après compactage, une bonne cohésion du produit, c'est-à-dire que le produit compacté ne s'effrite pas facilement.

On a de plus constaté que les dérivés selon l'invention permettent également de conférer à la composition cosmétique les comprenant des qualités d'étalement et d'adhésion à la peau particulièrement intéressantes, ainsi qu'un toucher onctueux et agréable.

Plus précisément, l'invention a pour objet un dérivé de l'arginine de formule (I) :

R - O - CO - NH - CH(COOH) - (CH₂)₃ - NH - C(NH) - NH₂

dans laquelle R représente de préférence un radical alkyle saturé, linéaire ou ramifié ayant de 8 à 22 atomes de carbone,
les sels des composés de formule (I), leurs isomères optiques de configuration D ou L et leurs mélanges.

Le radical R peut représenter un radical alkyle linéaire ou ramifié ayant de 8 à 16 atomes de carbone.
Parmi les dérivés selon l'invention, on peut citer la N^{α}-dodécyloxycarbonyl-L-arginine, la N^{α}-2-éthylhexyloxycarbonyl-L-arginine, la N^{α}-décyloxycarbonyl-L-arginine et la N^{α}-hexadécyloxycarbonyl-L-arginine.

Les sels des dérivés selon l'invention peuvent être choisis parmi les sels de cations inorganiques monovalents, tels que ceux de sodium, ou divalents tels que ceux de zinc ou de cuivre.
Les sels peuvent être aussi choisis parmi les sels de cations organiques tels que ceux d'aminopropanediol, de trishydroxyaminométhane, de glucamine et de N-méthylglucamine.

Les dérivés selon l'invention peuvent se présenter sous forme solide ayant une taille des particules comprise entre 10 - 500 000 nm, de préférence comprise entre 100 - 25000 nm.
Ces dérivés sont généralement insolubles dans les huiles et dans les solutions aqueuses dont le pH est compris entre 5 et 8.
Ils présentent généralement un point de fusion élevé, supérieur à 120°C.

La composition selon l'invention comprenant lesdits dérivés peut se présenter sous diverses formes telles que des dispersions, des lotions éventuellement épaissies ou gélifiées, des poudres éventuellement 〈〈 compactées 〉〉, des laits, des crèmes, des stick, des mousses ou des sprays lorsqu'elle est conditionnée en aérosol, des émulsions huile-dans-eau ou eau-dans-huile, de dispersions liposomiales ou encore de préparations solides.
Les dérivés selon l'invention peuvent être compris dans la composition en une proportion allant de 0,05% à 80% en poids, de préférence en une proportion de 0,5 à 30% en poids par rapport au poids total de la composition.
Les dérivés selon l'invention peuvent être présents dans la composition sous forme libre et'ou sous forme d'une association avec des particules substrats qu'ils enrobent.

Outre le dérivé selon l'invention, la composition peut aussi comprendre au moins un additif choisi dans le groupe constitué par les agents tensioactifs, les corps gras, les solvants organiques, les silicones, les épaississants, les adoucissants, les filtres solaires, les agents de traitement, les agents anti-mousse, les agents hydratants, les parfums, les conservateurs, les agents antioxydants, les séquestrants, les agents de sapidité, les agents alcalinisants ou acidifiants, les charges et les pigments.
Parmi les corps gras utilisables dans la composition selon l'invention, on peut citer les huiles, les cires, les acides gras, les alcools gras et/ou leur mélange. Les huiles peuvent être d'origine animale, végétale, minérale ou de synthèse. On peut citer en particulier l'huile de palme hydrogénée, l'huile de ricin hydrogénée, l'huile de vaseline, l'huile de paraffine, l'huile de purcellin. Les cires peuvent être d'origine animale, végétale, minérale ou de synthèse. On peut citer en particulier la cire d'abeille, la cire de montan, la cire de Carnauba, la cire de candelilla, la cire de canna à sucre, la cire du Japon, l'ozokérite, les cires microcristallines, la cire de paraffine, la cire de lanoline, la cire de lanoline hydrogénée et la cire de lanoline acétylée.

Parmi les particules pouvant être enrobées par les dérivés selon l'invention, on peut citer notamment les pigments, les charges particulaires et les microsphères telles que les microsphères creuses de copolymères de chlorure de vinylidène/acrylonitrile. Plus particulièrement, on peut citer, parmi les charges, les charges insolubles éventuellement colorées telles que des nanopigments d'oxydes métalliques comme les oxydes de titane, de zinc, de fer, de manganèse, de césium et/ou de zirconium.

La composition selon l'invention peut se présenter sous forme de compositions de maquillage telles que des fonds de teint, des crèmes teintées, des mascaras, des fards à joues et à paupières, des rouges à lèvres et des vernis à ongles.

Selon une forme particulière de réalisation, elle peut se présenter sous forme dite compacte, le dérivé selon l'invention facilitant le compactage des ingrédients desdites compositions. Parmi ces compositions compactes, on peut citer notamment des fonds de teint, des fards à joues ou à paupières, et les rouges à lèvres.

La composition selon l'invention peut également se présenter sous forme d'une composition pharmaceutique ou hygiénique telles que des pâtes dentifrices, des compositions exfoliantes, des poudres pour le corps ou pour bébés et des poudres antitranspirantes, voire sous forme d'une composition alimentaire.

L'invention a également pour objet un procédé de préparation de dérivé de formule (I), consistant :
- à faire réagir en milieu aqueux et à pH basique, de l'arginine ou l'un de ses sels, de configuration connue, avec un composé de formule R-O-CO-X, dans laquelle R représente un radical alkyle saturé, linéaire ou ramifié ayant de 8 à 22 atomes de carbone, et X est choisi dans le groupe constitué par un atome de chlore, un radical chlorométhyle et un radical imidazolyle, puis
- éventuellement à purifier le composé obtenu.

Le composé de formule R-O-CO-X peut être ajouté sans solvant.
Le pH basique du milieu réactionnel est de préférence compris entre 8 et 14.

On va maintenant donner à titre illustratif des exemples de préparation de dérivés d'arginine selon l'invention, ainsi qu'un exemple de composition comprenant un tel dérivé.

### EXEMPLE 1 : Préparation de la N^{α}-dodécyloxycarbonyl-L-arginine

Dans un ballon tricol de 500 ml, 10 g de L-arginine sont mis en solution dans 100 ml d'eau puis additionnés de 50 ml de THF.
14,3 g de chloroformiate de dodécyle est additionné goutte à goutte à la solution de L-arginine tandis que le pH apparent est maintenu constant par l'ajout de soude. Après 16 heures sous vive agitation, le milieu réactionnel est refroidi à 0°C et additionné de 400 ml d'acétone glacée. Le précipité obtenu est filtré sur verre fritté, lavé et séché sous pression réduite avant d'être recristallisé dans l'éthanol absolu.
On obtient 12,6 g (rendement 57%) de produit.

L'analyse chimique donne les caractéristiques suivantes :
- Fusion : T>140°C (bankofler)
- Poids moléculaire : 386,539
- Analyse élémentaire : C19H38N4O4

| | C | H | N | O |
|---|---|---|---|---|
| % calculé | 59,04 | 9,91 | 14,49 | 16,56 |
| % mesuré | 58,57 | 10,00 | 14,32 | 16,96 |

- RMN ¹H : à 400MHz dans le DMSO d₆: 0,86 (t, 3H) ; 1,43 et 1,45 à 1,63 (m, 24H); 3,03 (t, 2H) ; 3,67 (m, 1H), 3,90 (t, 2H) ; 6,2 (m, 1H) ; 7,9 (m, 5H)

### EXEMPLE 2 : Préparation de la N^{α}-éthylhexyloxycarbonyl-L-arginine

Dans un ballon tricol de 500 ml, 10 g de L-arginine sont mis en solution dans 100 ml d'eau puis additionné de 50 ml de THF.
11,05 g de chloroformiate de 2-éthylhéxyle est additionné goutte à goutte à la solution de L-arginine tandis que le pH apparent est maintenu constant par l'ajout de soude. Après 16 heures sous vive agitation, le milieu réactionnel est refroidi à 0°C et additionné de 100 ml d'acétone glacée. L'arginine résiduelle est séparée par filtration sur verre fritté tandis que le filtrat est concentré sous pression réduite en présence d'isopropanol. Le résidu est purifié plusieurs fois par solubilisation dans le minimum d'eau suivie de la précipitation des sels par l'isopropanol, filtration et concentration de filtrat.
On obtient 18,8 g (rendement voisin de 99%) de produit sous forme de solide pulvérulent.

L'analyse chimique donne les caractéristiques suivantes :
- Fusion : T>120°C
- Poids moléculaire : 330,431
- Analyse élémentaire : C15H30N4O4, H2O

| | C | H | N | O |
|---|---|---|---|---|
| % calculé | 51,70 | 9,26 | 16,07 | 22,96 |
| % mesuré | 51,12 | 8,68 | 16,07 | 21,62 |

- RMN ¹H à 400 MHz dans le DMSO d₆ (δ en ppm) : 0,85 (t, 3H) ; 0,87 (t, 3H) ; 1,27 à 1,66 (m, 13H) ; 3,05 (t, 2H) ; 3,67 (m 1H) ; 3,84 (m, 2H) ; 6,2 (m, 1H) ; 7,6 (m, 4H) ; 9,1 (m, 1H).

### EXEMPLE 3 : Préparation de la N^{α}-décyloxycarbonyl-L-arginine

Dans un ballon tricol de 250 ml, 10 g de L-arginine sont mis en solution dans 100 ml d'eau puis additionné de 50 ml de THF.
12,6 g de chloroformiate de décyle est additionné goutte à goutte à la solution de L-arginine tandis que le pH apparent est maintenu constant par l'ajout de soude. Après 16 heures sous vive agitation, le milieu réactionnel hétérogène est refroidi à 5°C, additionné à 200 ml d'acétone glacée puis filtré sur verre fritté. Le précipité est lavé plusieurs fois par l'acétone, séché et recristallisé dans un mélange acétone/méthanol 2/3 avec filtration à chaud puis lavage par de l'acétone et séchage.
On obtient 9,2 g (rendement 44 %) de produit.

L'analyse chimique donne les caractéristiques suivantes :
- Fusion : T>134°C
- Poids moléculaire : 358,485
- Analyse élémentaire : C17H34N4O4

| | C | H | N | O |
|---|---|---|---|---|
| % calculé | 56,98 | 9,49 | 15,64 | 17,88 |
| % mesuré | 56,89 | 9,50 | 15,73 | 17,69 |

- RMN 1H à 250 MHz dans le DMSO d₆ (δ en ppm) : 0,86 (t, 3H) ; 1,25 à 1,64 (m, 20H) ; 3,02 (m, 2H) ; 3,65 (m, 1H) ; 3,89 (t, 2H) ; 6,35 (d, 1H) ; 7,1 à 8,1 (m, 4H) ; 9,33 (m, 1H)

### EXEMPLE 4 : Préparation de la N^{α}-héxadécyloxycarbonyl-L-arginine

Dans un ballon tricol de 250 ml, 10 g de L-arginine sont mis en solution dans 100 ml d'eau puis additionné de 50 ml de THF.
17,5 g de chloroformiate d'hexadécyle est additionné goutte à goutte à la solution de L-arginine tandis que le pH apparent est maintenu constant par l'ajout de soude. Après 16 heures sous vive agitation, le milieu réactionnel hétérogène est refroidi à 5 °C, additionné à 1 litre d'acétone glacée puis filtré sur verre fritté. Le précipité est lavé plusieurs fois par de l'acétone, séché et recristallisé dans l'éthanol avec filtration à chaud puis lavage et séchage.
On obtient 14,75 g (rendement 58%) de produit.

L'analyse chimique donne les caractéristiques suivantes :
- Fusion : T>130°C
- Poids moléculaire : 442,647
- Analyse élémentaire : C23H46N4O4, H2O

| | C | H | N | O |
|---|---|---|---|---|
| % calculé | 59,97 | 10,50 | 12,15 | 17,37 |
| % mesuré | 60,31 -60,59 | 10,04 - 10,19 | 11,61 | 16,40 - 16,50 |

- RMN ¹H à 500 MHz dans le DMSO d₆( δ en ppm) : 0,86 (t, 3H) ; 1,25 à 1,38 et 1,40 à 1,65 (m, 32H) ; 3,03 (dd, 2H) ; 3,66 (dd, 1H) ; 3,88 (t, 2H) ; 6,26 (d, 1H) ; 7,2 à 8,0 (m, 4H) ; 9,3 (m, 1H)

### EXEMPLE 5 : Préparation d'une poudre compactée

On prépare une poudre ayant la composition suivante :

| *Composition A :* | |
|---|---|
| - Talc | 38,4 g |
| - Oxychlorure de bismuth | 10 g |
| - Stéarate de zinc | 4 g |
| - Composé de l'exemple 4 | 20 g |
| - Poudre de Nylon | 20 g |

| *Composition B :* | |
|---|---|
| - Oxydes de fer | 1,6 g |
| - Huile de vaseline | 6 g |

La poudre est obtenue de la façon suivante : on broie la composition A dans un broyeur de type KENWOOD pendant environ 5 minutes sous faible agitation. On ajoute la composition B et on broie l'ensemble environ 2 minutes à la même vitesse, puis 3 minutes à une vitesse plus rapide. On tamise ensuite la préparation sur un tamis de 0,16 mm, puis on compacte ce mélange dans des coupelles.

On obtient une poudre compactée présentant une bonne adhésion, qui s'étale bien et de manière agréable sur la peau, tout en étant douce au toucher. La présence du composé de l'exemple 4 évite un effritement trop facile du produit compacté.

## Revendications

1. Dérivé de l'arginine à groupement N^{α}-alkyloxycarbonyle de formule (I) 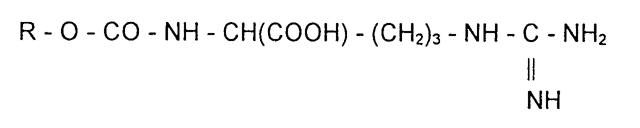 dans laquelle R représente un radical alkyle saturé, linéaire ou ramifié ayant de 8 à 22 atomes de carbone,
les sels des composés de formule (I), leurs isomères optiques de configuration D ou L et leurs mélanges.

2. Dérivé selon la revendication 1, caractérisé en ce que les sels sont choisis parmi les sels de cations inorganiques monovalents ou divalents, et les sels de cations organiques.

3. Dérivé selon l'une des revendications précédentes, caractérisé en ce que le radical R représente un radical alkyle, linéaire ou ramifié ayant de 8 à 16 atomes de carbone.

4. Dérivé selon l'une des revendications précédentes, caractérisé en ce qu'il est choisi parmi la Nα-dodécyloxycarbonyl-L-arginine, la Nα-2-ethylhexyloxycarbonyl-L-arginine, la Nα-décyloxycarbonyl-L-arginine et la Nα-hexadécyloxycarbonyl-L-arginine.

5. Dérivé selon l'une des revendications précédentes, caractérisé en ce qu'il a une taille de particules comprise entre 10 nm et 500 000 nm.

6. Composition caractérisée en ce qu'elle comprend au moins un dérivé selon l'une des revendications 1 à 5.

7. Composition selon la revendication 6, caractérisée en ce que le dérivé de l'arginine est compris entre 0,05 et 80 % en poids par rapport au poids total de la composition.

8. Composition selon la revendication 7, caractérisée en ce que le dérivé de l'arginine est compris entre 0,5 et 30 % en poids par rapport au poids total de la composition.

9. Composition selon l'une des revendications 6 à 8, caractérisée en ce qu'elle comprend le dérivé d'arginine sous forme libre et/ou sous forme d'une association avec des particules substrats qu'il enrobe.

10. Composition selon la revendication 9, dans laquelle les particules substrats sont choisies parmi les pigments, les charges particulaires et les microsphères telles que les microsphères creuses de copolymères de chlorure de vinylidène/acrylonitrile.

11. Composition selon l'une des revendications 6 à 10, se présentant sous forme d'une composition cosmétique, pharmaceutique, hygiénique ou alimentaire.

12. Composition selon l'une des revendications 6 à 11, se présentant sous forme d'un fond de teint, mascara, fard à joues et à paupières, rouge à lèvres, vernis à ongles, composition exfoliante, pâte dentifrice, poudre pour le corps ou pour bébés et/ou poudre antitranspirante.

13. Procédé de préparation d'un dérivé selon l'une des revendications 1 à 5, caractérisé en ce qu'il consiste à faire réagir en milieu aqueux et à pH basique, de l'arginine ou l'un de ses sels, de configuration connue, avec un composé de formule R - O - CO - X, dans laquelle R représente un radical alkyle saturé linéaire ou ramifié ayant de 8 à 22 atomes de carbone, et X est choisi dans le groupe constitué par un atome de chlore, un radical chlorométhyle et un radical imidazolyle.

14. Utilisation d'au moins un dérivé selon l'une des revendication 1 à 5, en tant que revêtement de particules substrat.

15. Utilisation d'au moins un dérivé selon l'une des revendications 1 à 5, pour faciliter le compactage de compositions comprenant des particules.

## Claims

1. Arginine derivative containing an N^{α}-alkyloxycarbonyl group of formula (I) 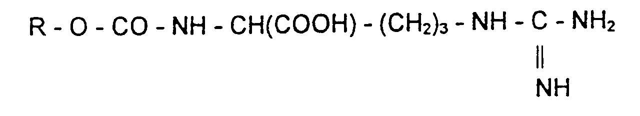 in which R represents a linear or branched saturated alkyl radical having from 8 to 22 carbon atoms,
the salts of the compounds of formula (I), their optical isomers of D or L configuration, and their mixtures.

2. Derivative according to Claim 1, characterized in that the salts are chosen from the salts of monovalent or divalent inorganic cations and the salts of organic cations.

3. Derivative according to one of the preceding claims, characterized in that the R radical represents a linear or branched alkyl radical having from 8 to 16 carbon atoms.

4. Derivative according to one of the preceding claims, characterized in that it is chosen from N^{α}-dodecyloxycarbonyl-L-arginine, N^{α}-2-ethylhexyloxycarbonyl-L-arginine, N^{α}-decyloxycarbonyl-L-arginine and N^{α}-hexadecyloxycarbonyl-L-arginine.

5. Derivative according to one of the preceding claims, characterized in that it has a particle size of between 10 nm and 500,000 nm.

6. Composition, characterized in that it comprises at least one derivative according to one of Claims 1 to 5.

7. Composition according to Claim 6, characterized in that the arginine derivative is between 0.05 and 80 % by weight with respect to the total weight of the composition.

8. Composition according to Claim 7, characterized in that the arginine derivative is between 0.5 and 30 % by weight with respect to the total weight of the composition.

9. Composition according to one of Claims 6 to 8, characterized in that it comprises the arginine derivative in the free form and/or in the form of a combination with substrate particles which it coats.

10. Composition according to Claim 9, in which the substrate particles are chosen from pigments, particulate fillers and microspheres such as hollow vinylidene chloride/acrylonitrile copolymer microspheres.

11. Composition according to one of Claims 6 to 10, which is provided in the form of a cosmetic, pharmaceutical, hygiene or food composition.

12. Composition according to one of Claims 6 to 11, which is provided in the form of a foundation, mascara, blusher, eyeshadow, lipstick, nail varnish, exfoliative composition, toothpaste, powder for the body or for babies, and/or anti-perspirant powder.

13. Process for the preparation of a derivative according to one of Claims 1 to 5, characterized in that it consists in reacting, in aqueous medium and at basic pH, arginine or one of its salts, of known configuration, with a compound of formula R-O-CO-X in which R represents a linear or branched saturated alkyl radical having from 8 to 22 carbon atoms and X is chosen from the group consisting of a chlorine atom, a chloromethyl radical and an imidazolyl radical.

14. Use of at least one derivative according to one of Claims 1 to 5, as coating for substrate particles.

15. Use of at least one derivative according to one of Claims 1 to 5, for facilitating compaction of compositions comprising particles.

## Patentansprüche

1. Argininderivate mit einer N^{α}-Alkyloxycarbonylgruppe der Formel (I) 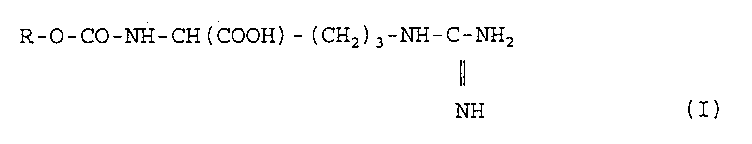 worin R eine gesättigte, lineare oder verzweigte Alkylgruppe mit 8 bis 22 Kohlenstoffatomen bedeutet, die Salze der Verbindungen der Formel (I), ihre optischen Isomere mit D- oder L-Konfiguration und deren Gemische.

2. Derivate nach Anspruch 1, dadurch gekennzeichnet, daß die Salze ausgewählt sind unter Salzen von einwertigen oder zweiwertigen anorganischen Kationen und Salzen von organischen Kationen.

3. Derivate nach einem der vorhergehenden Ansprüche, dadurch gekennzeichnet, daß die Gruppe R eine lineare oder verzweigte Alkylgruppe mit 8 bis 16 Kohlenstoffatomen bedeutet.

4. Derivate nach einem der vorhergehenden Ansprüche, dadurch gekennzeichnet, daß sie unter N^{α}-Dodecyloxycarbonyl-L-arginin, N^{α}-2-Ethylhexyloxycarbonyl-L-arginin, N^{α}-Decyloxycarbonyl-L-arginin und N^{α}-Hexadecyloxycarbonyl-L-arginin ausgewählt sind.

5. Derivate nach einer der vorhergehenden Ansprüche, dadurch gekennzeichnet, daß sie eine Partikelgröße im Bereich von 10 nm bis 500.000 nm aufweisen.

6. Zusammensetzung, dadurch gekennzeichnet, daß sie mindestens ein Derivat nach einem der Ansprüche 1 bis 5 enthält.

7. Zusammensetzung nach Anspruch 6, dadurch gekennzeichnet, daß das Argininderivat in Anteilen von 0,05 bis 80 Gew.-%, bezogen auf das Gesamtgewicht der Zusammensetzung, enthalten ist.

8. Zusammensetzung nach Anspruch 7, dadurch gekennzeichnet, daß das Argininderivat in Anteilen von 0,5 bis 30 Gew.-%, bezogen auf das Gesamtgewicht der Zusammensetzung, enthalten ist.

9. Zusammensetzung nach einem der Ansprüche 6 bis 8, dadurch gekennzeichnet, daß sie das Argininderivat in freier Form und/oder in Form einer Kombination mit Substratpartikeln, die es umhüllt, enthält.

10. Zusammensetzung nach Anspruch 9, in der die Substratpartikel ausgewählt sind unter Pigmenten, speziellen Füllstoffen und Mikrokügelchen, wie hohlen Mikrokügelchen von Vinylidenchlorid-Acrylnitril-Copolymeren.

11. Zusammensetzung nach einem der Ansprüche 6 bis 10, die in Form einer kosmetischen, pharmazeutischen Zusammensetzung oder Zusammensetzung zur Hygiene oder einer Lebensmittelzusammensetzung vorliegt.

12. Zusammensetzung nach einem der Ansprüche 6 bis 11, die als Make up, Mascara, Wangenrouge, Lidschatten, Lippenstift, Nagellack, Peelingzusammensetzung, Zahnpasta, Körperpuder oder Babypuder und/oder Antitranspirantpulver vorliegt.

13. Verfahren zur Herstellung eines Derivats nach einem der Ansprüche 1 bis 5, dadurch gekennzeichnet, daß es darin besteht, Arginin oder eines seiner Salze bekannter Konfiguration mit einer Verbindung der Formel R-O-CO-X, worin R eine gesättigte lineare oder verzweigte Alkylgruppe mit 8 bis 22 Kohlenstoffatomen bedeutet und X unter einem Chloratom, einer Chlormethylgruppe und einer Imidazolylgruppe ausgewählt ist, in wäßrigem Medium bei einem basischen pH-Wert umzusetzen.

14. Verwendung mindestens eines Derivats nach einem der Ansprüche 1 bis 5 als Überzug von Substratpartikeln.

15. Verwendung mindestens eines Derivats nach einem der Ansprüche 1 bis 5 zu Erleichterung der Verdichtung von Zusammensetzungen, die Partikel enthalten.
